# EUROPEAN PATENT APPLICATION

(11) **EP 4 725 945 A1**
(43) Date of publication of application: **15.04.2026**
(21) Application number: 24818758.5
(22) Date of filing: 07.06.2024
(51) Int. Cl.: C07D 401/12, C07D 471/04, C07D 471/14, C07D 498/14

(54) **PREPARATION METHOD FOR FORMAMIDE COMPOUNDS**

(30) Priority: 08.06.2023 CN 202310675632
(71) Applicant: Shanghai Senhui Medicine Co., Ltd., Shanghai 201203 (CN); Shanghai Shengdi Pharmaceutical Co., Ltd., Shanghai 201210 (CN); Jiangsu Hengrui Pharmaceuticals Co., Ltd., Lianyungang, Jiangsu 222047 (CN)
(72) Inventor: JIANG, Wei, Shanghai 201203 (CN); PENG, Yinglong, Shanghai 201203 (CN); LI, Bei, Shanghai 201203 (CN); YU, Qiang, Shanghai 201203 (CN); HUANG, Jian, Shanghai 201203 (CN)
(74) Representative: Regimbeau
(86) International application number: PCT/CN2024/097931
(87) International publication number: WO 2024/251227

(57) **Abstract**

The present disclosure relates to a preparation method for formamide compounds. In particular, the present disclosure relates to a preparation method for compounds shown as formula 7a or pharmaceutically acceptable salts thereof. The preparation method has a high yield and is suitable for industrial production.

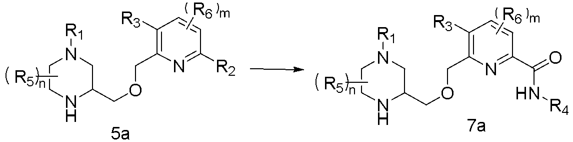

## Description

### Technical Field

The present disclosure belongs to the field of medicine and relates to a preparation method for preparing a formamide compound.

### Background Art

Poly(ADP-ribose)polymerase 1 (PARP1) was first reported more than 50 years ago, and since then, it has been gradually discovered to play an important role in DNA repair, maintenance of genome integrity, and regulation of various metabolic and signalling processes. PARP1 can catalyse the transfer of an ADP-ribose residue from NAD+ to a target substrate, thereby constructing a poly(ADP-ribose) chain (referred to as the PAR chain). The formation and clearance of PAR chains occur in nearly all eukaryotic cells.

ADP-ribosylation is a post-translational modification of proteins that is widely present in various physiological and pathological processes. It refers to the binding of one or more ADP-ribose units to specific sites on proteins under the catalysis of enzymes. PARP1 is the first member of the PARP superfamily, which consists of proteins homologous to PARP1. Currently, there are 17 members in this superfamily, among which 4 members (PARP1, PARP2, PARP5A and PARP5B) can synthesise PAR chains. Most other enzymes in this superfamily can only construct single ADP-ribose units and are therefore classified as mono(ADP-ribosyl)ases (MARs).

WO 2022247816 A discloses the following synthetic route for formamide compounds. Compound 8 was prepared using compound 8a as the starting material via a six-step reaction with an overall yield of 2.7%.

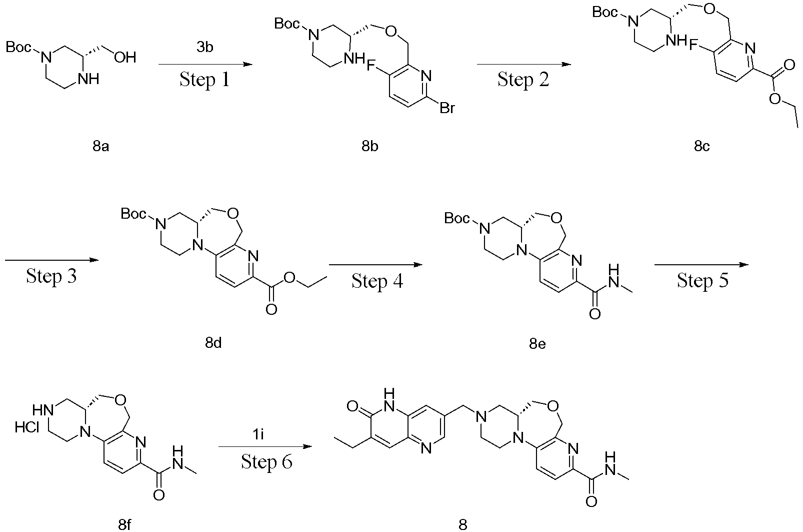

### Summary of the Invention

In a first aspect, the present disclosure provides a method for preparing a compound shown as formula 7a or a pharmaceutically acceptable salt thereof, which has the advantages of low dosage of reaction catalyst, improved yield, and controllable reaction temperature. The method comprises reacting a compound shown as formula 5a or a pharmaceutically acceptable salt thereof in the presence of carbon monoxide, a palladium catalyst and R₄-NH₂ to afford the compound shown as formula 7a or the pharmaceutically acceptable salt thereof, 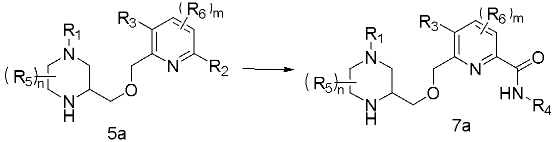
wherein R₁ is an amino protecting group;
R₂ is halogen;
R₃ is halogen;
R₄ is selected from hydrogen, C₁₋₆ alkyl, hydroxylated C₁₋₆ alkyl, or halogenated C₁₋₆ alkyl;
each R₅ is independently selected from halogen, C₁₋₆ alkyl, hydroxylated C₁₋₆ alkyl, halogenated C₁₋₆ alkyl, C₁₋₆ alkoxy, hydroxylated C₁₋₆ alkoxy, halogenated C₁₋₆ alkoxy, hydroxyl, cyano, or oxo;
each R₆ is independently selected from halogen, C₁₋₆ alkyl, hydroxylated C₁₋₆ alkyl, halogenated C₁₋₆ alkyl, C₁₋₆ alkoxy, hydroxylated C₁₋₆ alkoxy, halogenated C₁₋₆ alkoxy, hydroxyl, or cyano;
n is an integer selected from 0-6;
m is an integer selected from 0-2.

In some embodiments, according to the preparation method of the first aspect, n is selected from 0, 1, 2, 3, or 4, preferably n is selected from 0, 1, or 2, and more preferably n is selected from 0, or 1.

In some embodiments, according to the preparation method of the first aspect, m is selected from 0, or 1.

In some embodiments, according to the preparation method of the first aspect, R₂ is selected from fluorine, chlorine, bromine or iodine, and preferably R₂ is bromine.

In some embodiments, according to the preparation method of the first aspect, R₃ is selected from fluorine, chlorine, bromine or iodine, and preferably R₃ is chlorine.

In some embodiments, according to the preparation method of the first aspect, the palladium catalyst is a divalent palladium catalyst.

In some embodiments, according to the preparation method of the first aspect, the palladium catalyst is selected from palladium acetate, palladium dichloride, tris(dibenzylideneacetone)dipalladium, bis(dibenzylideneacetone)palladium, palladium trifluoroacetate, palladium pivalate, tetrakis(triphenylphosphine)palladium, bis(triphenylphosphine)palladium chloride, bis(benzonitrile)palladium chloride, or bis(acetonitrile)dichloropalladium.

In some embodiments, according to the preparation method of the first aspect, the palladium catalyst is palladium acetate.

In some embodiments, according to the preparation method of the first aspect, the molar ratio of the compound shown as formula 5a to the palladium catalyst is 1 : 0.1-1 : 0.0001; preferably 1 : 0.01-1 : 0.0005; more preferably 1 : 0.01-1 : 0.001; and most preferably 1 : 0.005.

In some embodiments, according to the preparation method of the first aspect, the method comprises reacting the compound shown as formula 5a or the pharmaceutically acceptable salt thereof in the presence of a phosphine ligand to afford the compound shown as formula 7a or the pharmaceutically acceptable salt thereof.

In some embodiments, according to the preparation method of the first aspect, the phosphine ligand is selected from triphenylphosphine, tri(o-tolyl)phosphine, tri-tert-butylphosphine, tricyclohexylphosphine, 2-(dicyclohexylphosphino)biphenyl, 2-(di-tert-butylphosphino)biphenyl, 2-(dicyclohexylphosphino)-2'-methylbiphenyl, 2-(di-tert-butylphosphino)-2'-methylbiphenyl, 1,1'-bis(diphenylphosphino)ferrocene, (±)-2,2'-bis(diphenylphosphino)-1,1'-binaphthalene, 4,5-bis(diphenylphosphino)-9,9-dimethylxanthene, bis(2-diphenylphosphinophenyl)ether, 2-dicyclohexylphosphino-2',6'-diisopropoxybiphenyl, 2-dicyclohexylphosphino-2',4',6'-triisopropylbiphenyl, 2-(dicyclohexylphosphino)biphenyl, 2-(di-tert-butylphosphino)biphenyl, 2-dicyclohexylphosphino-2'-(N,N-dimethylamino)biphenyl, 2-dicyclohexylphosphino-2',6'-dimethoxybiphenyl, 2-(di-tert-butylphosphino)-3,6-dimethoxy-2'-4'-6'-triisopropyl-1,1'-biphenyl, dicyclohexyl(2',4',6'-triisopropyl-3,6-dimethoxy-[1,1'-biphenyl]-2-yl)phosphine, [(4-(N,N-dimethylamino)phenyl]di-tert-butylphosphine, 2-di-tert-butylphosphino-3,4,5,6-tetramethyl-2',4',6'-triisopropylbiphenyl, 1,2-bis(diphenylphosphino)ethane, 1,3-bis(diphenylphosphino)propane, or 1,4-bis(diphenylphosphino)butane.

In some embodiments, according to the preparation method of the first aspect, the phosphine ligand is 1,1'-bis(diphenylphosphino)ferrocene.

In some embodiments, according to the preparation method of the first aspect, the molar ratio of the compound shown as formula 5a to the phosphine ligand is 1 : 0.1 - 1 : 0.0001; preferably 1 : 0.01 - 1 : 0.0005; more preferably 1 : 0.01 - 1 : 0.001; and most preferably 1 : 0.005.

In some embodiments, according to the preparation method of the first aspect, the method comprises reacting the compound shown as formula 5a or the pharmaceutically acceptable salt thereof in the presence of a base to afford the compound shown as formula 7a or the pharmaceutically acceptable salt thereof.

In some embodiments, according to the preparation method of the first aspect, the base is selected from sodium hydroxide, sodium carbonate, caesium carbonate, triethylamine, sodium acetate or pyridine.

In some embodiments, according to the preparation method of the first aspect, the base is sodium acetate.

In some embodiments, according to the preparation method of the first aspect, the molar ratio of the compound shown as formula 5a to the base is 1 : 5 - 1 : 0.5, preferably 1 : 5 - 1 : 1, more preferably, and most preferably 1 : 2.

In some embodiments, according to the preparation method of the first aspect, the preparation method is carried out in a solvent, and the solvent includes, but is not limited to, one or more of a halogenated hydrocarbon solvent, a nitrile solvent, an amide solvent, and a sulfoxide solvent.

In some embodiments, according to the preparation method of the first aspect, the preparation method is carried out in a solvent, and the solvent includes, but is not limited to, one or more of dichloromethane, methanol, ethanol, N,N-dimethylformamide, and N,N-dimethylacetamide.

In some embodiments, according to the preparation method of the first aspect, the preparation method is carried out in a solvent, and the solvent is methanol and N,N-dimethylformamide.

In some embodiments, according to the preparation method of the first aspect, the R₄ is selected from C₁₋₆ alkyl, hydroxylated C₁₋₆ alkyl, or halogenated C₁₋₆ alkyl.

In some embodiments, according to the preparation method of the first aspect, the R₄ is selected from methyl, ethyl, propyl, butyl, hydroxymethyl, hydroxyethyl, hydroxypropyl, hydroxybutyl, halomethyl, haloethyl, halopropyl, or halobutyl.

In some embodiments, according to the preparation method of the first aspect, the R₄ is methyl.

In some embodiments, according to the preparation method of the first aspect, the R₁ is selected from C₁-C₆ alkylacyl, C₆-C₁₀ arylacyl, C₁-C₆ alkylsulfonyl, C₆-C₁₀ arylsulfonyl, C₁-C₆ alkoxycarbonyl, C₆-C₁₀ aryloxycarbonyl, or substituted or unsubstituted C₇-C₁₂ benzyl.

In some embodiments, according to the preparation method of the first aspect, the R₁ is selected from formyl, acetyl, benzoyl, tertbutoxycarbonyl (Boc), benzyloxycarbonyl (Cbz), 2,4-dimethoxybenzyl (DMB), p-methoxybenzyl (PMB) or benzyl (Bn).

In some embodiments, according to the preparation method of the first aspect, the R₁ is tertbutoxycarbonyl (Boc).

In some embodiments, according to the preparation method of the first aspect, the preparation method can comprise reacting a compound shown as formula 5b or a pharmaceutically acceptable salt thereof to afford a compound shown as formula 7b or a pharmaceutically acceptable salt thereof,

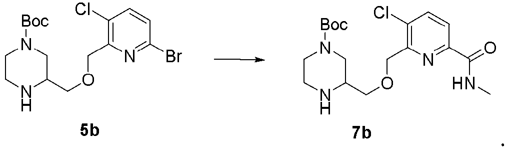

In some embodiments, according to the preparation method of the first aspect, the preparation method can comprise reacting a compound shown as formula 5 or a pharmaceutically acceptable salt thereof to afford a compound shown as formula 7 or a pharmaceutically acceptable salt thereof,

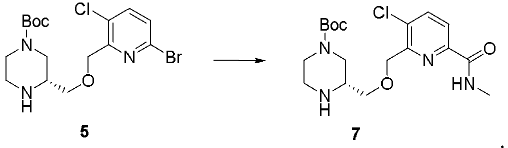

In some embodiments, according to the preparation method of the first aspect, the preparation method further comprises adding an organic acid after the reaction is completed, wherein the organic acid is selected from formic acid, acetic acid, and p-toluenesulfonic acid.

In some embodiments, according to the preparation method of the first aspect, the preparation method further comprises adding an organic acid after the reaction is completed, wherein the organic acid is p-toluenesulfonic acid. By adding the organic acid to form a corresponding salt, the crystallinity of the product can be increased, and impurities generated during the reaction, catalyst ligands, etc. can be removed, thereby facilitating post-processing and purification. Conversion from the salt form to the free form can be performed by methods well known to those skilled in the art, such as using an appropriate base, including but not limited to sodium bicarbonate, sodium carbonate, sodium hydroxide, potassium bicarbonate, potassium carbonate, potassium hydroxide, etc.

In a second aspect, the present disclosure further provides a method for preparing a compound shown as formula 8a or a pharmaceutically acceptable salt thereof, comprising reacting a compound shown as formula 7a or a pharmaceutically acceptable salt thereof to afford the compound shown as formula 8a or the pharmaceutically acceptable salt thereof, 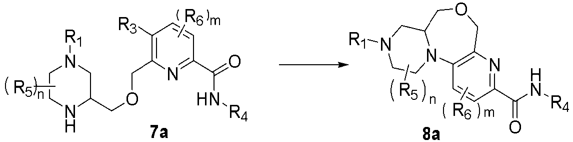 wherein, R₁, R₃, R₄, R₅, R₆, n and m are as defined in the first aspect.

In some embodiments, according to the preparation method of the first or second aspect, each R₅ is independently selected from C₁₋₆ alkyl, hydroxylated C₁₋₆ alkyl, halogenated C₁₋₆ alkyl, C₁₋₆ alkoxy, hydroxylated C₁₋₆ alkoxy, or halogenated C₁₋₆ alkoxy.

In some embodiments, according to the preparation method of the first or second aspect, each R₅ is independently selected from halogen, hydroxyl, cyano, or oxo.

In some embodiments, according to the preparation method of the first or second aspect, each R₅ is independently selected from C₁₋₆ alkyl, hydroxylated C₁₋₆ alkyl, or halogenated C₁₋₆ alkyl.

In some embodiments, according to the preparation method of the first or second aspect, each R₆ is independently selected from halogen, hydroxyl, or cyano.

In some embodiments, according to the preparation method of the first or second aspect, each R₆ is independently selected from C₁₋₆ alkyl, hydroxylated C₁₋₆ alkyl, halogenated C₁₋₆ alkyl, C₁₋₆ alkoxy, hydroxylated C₁₋₆ alkoxy, or halogenated C₁₋₆ alkoxy.

In some embodiments, according to the preparation method of the first or second aspect, each R₆ is independently selected from C₁₋₆ alkyl, hydroxylated C₁₋₆ alkyl, or halogenated C₁₋₆ alkyl.

In some embodiments, according to the preparation method of the second aspect, the method comprises reacting a compound shown as formula 7b or a pharmaceutically acceptable salt thereof to afford a compound shown as formula 8b or a pharmaceutically acceptable salt thereof,

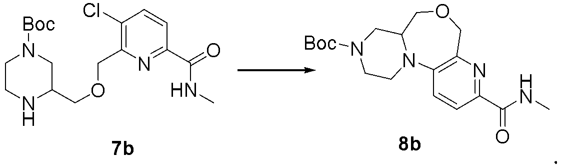

In some embodiments, according to the preparation method of the second aspect, the method comprises reacting a compound shown as formula 7 or a pharmaceutically acceptable salt thereof to afford a compound shown as formula 8 or a pharmaceutically acceptable salt thereof,

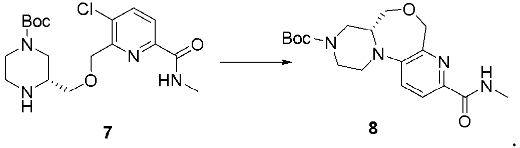

In some embodiments, according to the preparation method of the second aspect, the method comprises the preparation method for preparing the compound shown as formula 7a, 7b or 7 or the pharmaceutically acceptable salt thereof of the first aspect.

In some embodiments, according to the preparation method of the second aspect, the preparation method is reacted in the presence of a palladium catalyst, a phosphine ligand, and a base.

In some embodiments, according to the preparation method of the second aspect,
the palladium catalyst can be selected from tris(dibenzylideneacetone)dipalladium, bis(dibenzylideneacetone)palladium, palladium acetate, palladium chloride, bis(acetonitrile)dichloropalladium, bis(benzonitrile)palladium chloride, chloro(2-dicyclohexylphosphino-2',6'-di-isopropoxy-1,1'-biphenyl)(2-amino-1,1'-biphenyl-2-yl)palladium (II), chloro(2-dicyclohexylphosphino-2',6'-diisopropoxy-1,1'-biphenyl)[2- (2-aminoethylphenyl)] palladium (II) methyl tert-butyl ether complex, or methanesulfonato(2-dicyclohexylphosphino-2',6'-diisopropoxy-1,1'-biphenyl)(2'-amino-1,1'-biphenyl-2-yl)palladium (II),
the phosphine ligand can be selected from 2-dicyclohexylphosphino-2',6'-diisopropoxybiphenyl, tri-tert-butylphosphine, tricyclohexylphosphine, 2-(dicyclohexylphosphino)-2'-methylbiphenyl, 2-(di-tert-butylphosphino)-2'-methylbiphenyl, 1,1'-bis(diphenylphosphino)ferrocene, (±)-2,2'-bis(diphenylphosphino)-1,1'-binaphthalene, 4,5-bis(diphenylphosphino)-9,9-dimethylxanthene, bis(2-diphenylphosphinophenyl)ether, 2-dicyclohexylphosphino-2',4',6'-triisopropylbiphenyl, 2-(dicyclohexylphosphino)biphenyl, 2-(di-tert-butylphosphino)biphenyl, 2-dicyclohexylphosphino-2'-(N,N-dimethylamino)biphenyl, 2-dicyclohexylphosphino-2',6'-dimethoxybiphenyl, 2-(di-tert-butylphosphino)-3,6-dimethoxy-2'-4'-6'-triisopropyl-1,1'-biphenyl, dicyclohexyl(2',4',6'-triisopropyl-3,6-dimethoxy-[1,1'-biphenyl]-2-yl)phosphine, [(4-(N,N-dimethylamino)phenyl]di-tert-butylphosphine, or 2-di-tert-butylphosphino-3,4,5,6-tetramethyl-2',4',6'-triisopropylbiphenyl, and
the base can be selected from caesium carbonate, potassium carbonate, potassium phosphate, sodium tert-butoxide, sodium tert-pentoxide, potassium tert-butoxide, lithium hexamethyldisilazide, sodium hexamethyldisilazide, potassium hexamethyldisilazide, or caesium hydroxide.

In some embodiments, according to the preparation method of the second aspect, the molar ratio of the compound shown as formula 7a to the palladium catalyst is selected from 1 : 0.1 - 1 : 0.001, preferably the molar ratio is selected from 1 : 0.1 - 1 : 0.005, and more preferably the molar ratio is selected from 1 : 0.1 - 1 : 0.01; and
the molar ratio of the palladium catalyst to the phosphine ligand is selected from 1 : 10 - 1 : 0.1, preferably the molar ratio is selected from 1 : 5 - 1 : 0.1, and more preferably the molar ratio is selected from 1 : 5 - 1 : 1.

In some embodiments, according to the preparation method of the second aspect, the preparation method is carried out under inert gas atmosphere, preferably nitrogen atmosphere or argon atmosphere.

In some embodiments, according to the preparation method of the second aspect, the method comprises the step of reacting a compound shown as formula 5a or a pharmaceutically acceptable salt thereof to afford the compound shown as formula 7a or the pharmaceutically acceptable salt thereof.

In a third aspect, the present disclosure further provides a method for preparing a compound shown as formula 11a or a pharmaceutically acceptable salt thereof, comprising
(1) preparing the compound shown as formula 8a or the pharmaceutically acceptable salt thereof according to the preparation method of the second aspect; and
(2) using the compound shown as formula 8a or the pharmaceutically acceptable salt thereof to prepare the compound shown as formula 11a or the pharmaceutically acceptable salt thereof;

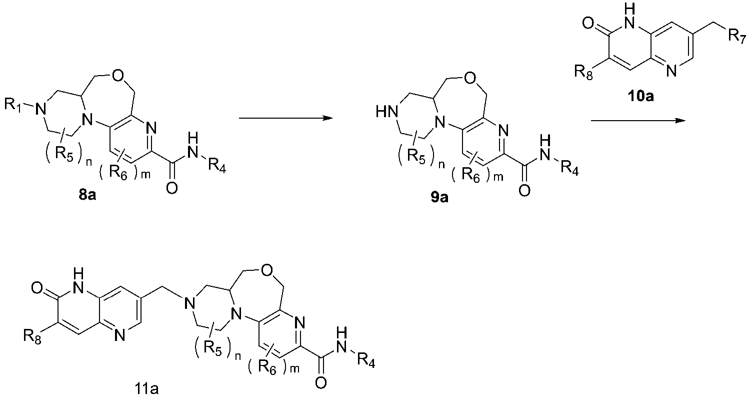

wherein, R₁, R₄, R₅, R₆, n and m are as defined in the first aspect;
R₇ is halogen;
R₈ is selected from hydrogen, C₁₋₆ alkyl, hydroxylated C₁₋₆ alkyl, or halogenated C₁₋₆ alkyl, and preferably the R₈ is selected from methyl, ethyl, propyl, butyl, hydroxymethyl, hydroxyethyl, hydroxypropyl, hydroxybutyl, halomethyl, haloethyl, halopropyl, or halobutyl.

In a fourth aspect, the present disclosure further provides a method for preparing a compound shown as formula 11b or a pharmaceutically acceptable salt thereof, comprising
(1) preparing the compound shown as formula 8b or the pharmaceutically acceptable salt thereof according to the preparation method of the second aspect; and
(2) using the compound shown as formula 8b or the pharmaceutically acceptable salt thereof to prepare the compound shown as formula 11b or the pharmaceutically acceptable salt thereof.

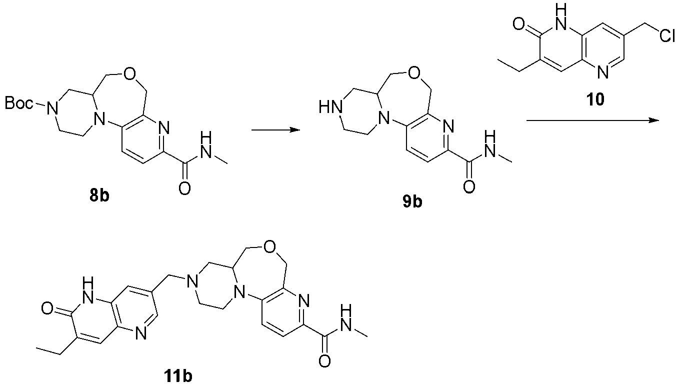

In a fifth aspect, the present disclosure further provides a method for preparing a compound shown as formula 11 or a pharmaceutically acceptable salt thereof, comprising
(1) preparing the compound shown as formula 8 or the pharmaceutically acceptable salt thereof according to the preparation method of the second aspect; and
(2) using the compound shown as formula 8 or the pharmaceutically acceptable salt thereof to prepare the compound shown as formula 11 or the pharmaceutically acceptable salt thereof;

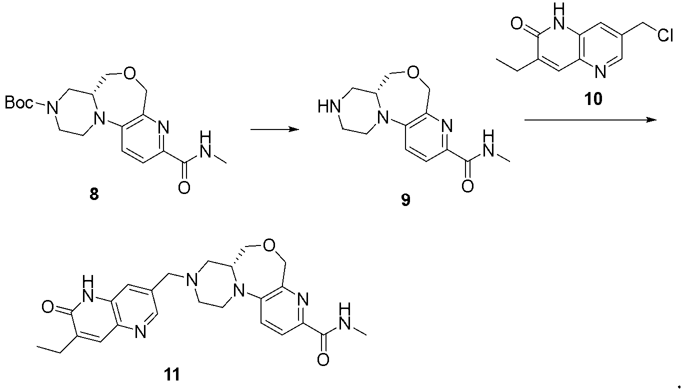

In some embodiments, for the compound shown as formula 11 or the pharmaceutically acceptable salt thereof, the pharmaceutically acceptable salt is a fumaric acid salt.

In another aspect, the preparation method of the present disclosure further comprises one or more steps of filtration, concentration, purification by column chromatography and drying.

The terms "to form" and "converting into" of the present disclosure do not specifically mean that the conversion reaction between two substrates is a single step, and can be a single step or multi-step reaction between two substrates. If the intermediate contains a protecting group, the intermediate is subjected to a step of removing the protecting agent, and then reacted with a corresponding substrate to obtain a corresponding target product.

The numerical values of the present disclosure are instrument measurements and have a certain degree of error. In general, plus or minus 10% is within a reasonable error range. Of course, the context in which the numerical value is used needs to be considered. For example, the error of the numerical value for the particle size of the active ingredient after measurement does not exceed plus or minus 10%, and can does not exceed plus or minus 9%, plus or minus 8%, plus or minus 7%, plus or minus 6%, plus or minus 5%, plus or minus 4%, plus or minus 3%, plus or minus 2% or plus or minus 1%, preferably plus or minus 5%.

The salts of the compounds of the present disclosure may be selected from inorganic salts or organic salts. The salts include acid addition salts and base addition salts. For example, the salts include salts formed by an acid-base reaction with a basic (amino) group, wherein the acid includes an organic or inorganic acid. In some embodiments, the salt of the compound shown as formula 7a is a p-toluenesulfonic acid salt. In some embodiments, the salt of the compound shown as formula 9 is a hydrochloride salt, and the salt of the compound shown as formula 11 is a fumaric acid salt.

In the chemical structures of the compounds of the present disclosure, a bond " 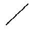 " represents an unspecified configuration; that is, if chiral isomers exist in the chemical structures, the bond " 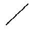 " may be " 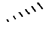 " or " 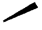 ", or both the configurations of " 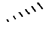 " and " 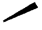 " are included simultaneously.

The term "alkyl" refers to a saturated aliphatic hydrocarbon group, including alkyl having 1 to 6 carbon atoms. Non-limiting examples include methyl, ethyl, n-propyl, and isopropyl. The alkyl may be optionally substituted or unsubstituted.

The term "halogen" refers to fluorine, chlorine, bromine or iodine.

The term "amino" refers to -NH₂.

The term "hydroxyl" refers to -OH group.

The term "mercapto" refers to -SH group.

The term "carboxyl" refers to -C(O)OH.

When the functional groups of the present disclosure are substituted, the substituents are preferably one or more of the following groups, such as halogen, oxo, nitro, cyano, C₁-C₆ alkyl, C₁-C₆ alkoxy or C₁-C₆ cycloalkyl.

The "amino protecting group" is a suitable group known in the art for amino protection, see the amino protecting group in the document ("Protective Groups in Organic Synthesis", 5Th. Ed. T. W. Greene & P. G. M. Wuts). Preferably, the amino protecting group may be (C₁-C₁₀ alkyl or aryl)acyl, such as formyl, acetyl, and benzoyl. The amino protecting group may be (C₁-C₆ alkyl or C₁-C₁₀ aryl)sulfonyl. The amino protecting group may also be (C₁-C₆ alkoxy or C₁-C₁₀ aryloxy)carbonyl, such as Boc or Cbz. The amino protecting group may also be substituted or unsubstituted alkyl, such as trityl (Tr), 2,4-dimethoxybenzyl (DMB), p-methoxybenzyl (PMB) or benzyl (Bn).

### Detailed Description of Embodiments

The present disclosure will be further described below in conjunction with examples, but these examples are not intended to limit the scope of the present disclosure.

### Example

The structures of the compounds are determined by nuclear magnetic resonance (NMR) or /and mass spectrometry (MS). The NMR shift (δ) is given in the unit of 10⁻⁶ (ppm). NMR is determined by Bruker AVANCE-400 nuclear magnetic resonance spectrometer or Bruker AVANCE NEO 500M; the solvents for determination are deuterated dimethyl sulfoxide (DMSO-d₆), deuterated chloroform (CDCl₃) and deuterated methanol (CD₃OD); and the internal standard is tetramethylsilane (TMS).

MS is determined by Agilent 1200/1290DAD-6110/6120Quadrupole MS liquid chromatography-mass spectrometer (manufacturer: Agilent, MS model: 6110/6120Quadrupole MS),
waters ACQuity UPLC-QD/SQD (manufacturer: waters, MS model: waters ACQuity Qda Detector/waters SQ Detector) and THERMO Ultimate 3000-Q Exactive (manufacturer: THERMO, MS model: THERMO Q Exactive).

High performance liquid chromatography (HPLC) analysis is carried out by Agilent HPLC 1200DAD, Agilent HPLC 1200VWD and Waters HPLC e2695-2489 liquid chromatographs.

Chiral HPLC analysis is determined by Agilent 1260 DAD high-performance liquid chromatograph.

Yantai Huanghai HSGF254 or Qingdao GF254 silica gel plate is used as a thin layer chromatography silica plate, and the silica gel plate for the thin layer chromatography (TLC) is of the specification of 0.15 mm-0.2 mm, and the specification when separating and purifying a product by thin layer chromatography is 0.4 mm-0.5 mm.

For silica gel column chromatography, Yantai Huanghai silica gel 200-300 mesh silica gel is generally used as a carrier.

Known starting materials in the present disclosure may be synthesised using or according to methods known in the art, or may be purchased from ABCR GmbH&Co.KG, Acros Organics, Aldrich Chemical Company, Accela ChemBio Inc., Chembee Chemicals, and other companies.

If there is no special instruction in the examples, reactions can be carried out under argon atmosphere or nitrogen atmosphere.

The argon atmosphere or nitrogen atmosphere means that the reaction bottle is connected to an argon or nitrogen balloon with a volume of about 1 L.

The hydrogen atmosphere means that the reaction bottle is connected to a hydrogen balloon with a volume of about 1 L.

Parr 3916EKX hydrogenator, Qinglan QL-500 hydrogen generator or HC2-SS hydrogenator are used for pressurized hydrogenation reaction.

Unless otherwise specified in the examples, a solution refers to an aqueous solution.

Unless otherwise indicated, the reaction temperature in the examples refers to room temperature, which is 20°C to 30°C.

The progress of the reaction in the examples is monitored by means of thin layer chromatography (TLC). The developing solvent used in the reaction, the eluent system of column chromatography used in the purification of compounds and the developing solvent system of thin layer chromatography include: A: dichloromethane/methanol system, wherein the volume ratio of the solvent is adjusted according to the polarity of compounds and can also be adjusted by adding a small amount of basic reagents such as triethylamine or acidic reagents such as acetic acid.

### Example 1 Preparation of (3, 6-dichloropyridin-2-yl)methanol (compound 2)

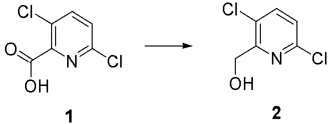

To a flask were added **compound 1** (57.6 g, 0.3 mol, 1.0 eq.) and ethylene glycol dimethyl ether (400 mL, 7 V), the mixture was cooled in ice water, and N-methylmorpholine (38.23 g, 0.378 mol, 1.26 eq.) and isobutyl chloroformate (51.22 g, 0.375 mol, 1.25 eq.) were dropwise added. The resulting mixture was stirred and filtered, and the filtrate was recorded as solution A. To another flask were added water (375 mL, 6.5 V), ethylene glycol dimethyl ether (375 mL, 6.5 V) and sodium borohydride (25.31 g, 0.669 mol, 2.23 eq.), solution A was dropwise added, and the mixture was stirred until the reaction was completed. Water and ethyl acetate were added, and the organic phase was separated, dried and concentrated to afford **compound 2** as a yellow oil (53.5 g, yield: 100%, purity: 96.4%).

### Example 2 Preparation of 6-bromo-2-(bromomethyl)-3-chloropyridine (compound 3)

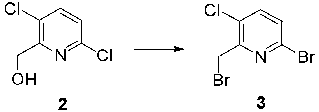

To a flask were added **compound 2** (52.0 g, 0.292 mol, 1.0 eq.) and 33% hydrobromic acid acetic acid solution (501.4 g, 2.05 mol, 7 eq.), the mixture was warmed to 105°C-110°C, and the reaction process was monitored by HPLC until the raw materials were converted completely. The reaction system was cooled to room temperature, water was added, and the mixture was filtered and dried to afford **compound 3** (75.6 g, yield: 90.7%, purity: 98.02%).

### Example 3 Preparation of (R) -tert-butyl 3-(((6-bromo-3-chloropyridin-2-yl)methoxy)methyl)piperazine-1-carboxylate (compound 5)

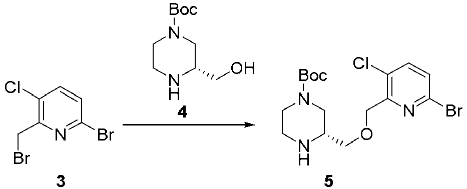

In a flask, **compound 3** (31.83 g, 147.18 mmol, 1.2 eq.) was dissolved with tetrahydrofuran (350 mL, 10 V), the mixture was cooled in ice water, and 60% sodium hydride (7.36 g, 183.98 mmol, 1.5 eq.) was added. The resulting mixture was stirred evenly, a solution of **compound 4** (35.00 g, 122.65 mmol, 1.0 eq.) in tetrahydrofuran (175 mL, 5 V) was dropwise added, and the mixture was stirred until the reaction was completed. Saturated aqueous ammonium chloride solution was added, and the resulting mixture was extracted with ethyl acetate and concentrated to afford **compound 5** (44.04 g, yield: 85.3%).

### Example 4 Preparation of (R) -tert-butyl 3-(((3-chloro-6-(methylcarbamyl)pyridin-2-yl)methoxy)methyl)piperazine-1-carboxylate 4-methylbenzenesulfonic acid salt (compound 6)

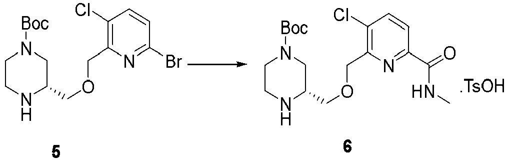

In a pressure bottle, **compound 5** (38.8 g, 92.22 mmol, 1.0 eq.) was dissolved with N,N-dimethylacetamide (194 mL, 5 V) and methanol (194 mL, 5 V), palladium acetate (104 mg, 0.461 mmol, 0.005 eq.), 1,1'-bis(diphenylphosphino)ferrocene (256 mg, 0.461 mmol, 0.005 eq.), sodium acetate(15.13 g, 184.4 mmol, 2.0 eq.) and 30% methylamine ethanol solution were added, and the mixture was stirred under carbon monoxide at 90°C-100°C until the reaction was completed. Saturated sodium bicarbonate solution was added, the mixture was extracted with ethyl acetate, and 4-methylbenzenesulfonic acid monohydrate was added to the organic phase. The resulting mixture was stirred, filtered and dried to afford **compound 6** (44.80 g, yield: 85%, purity: 98.3%).

### Example 5 Preparation of (R)-tert-butyl 3-(((3-chloro-6-(methylcarbamyl)pyridin-2-yl)methoxy)methyl)piperazine-1-carboxylate (compound 7)

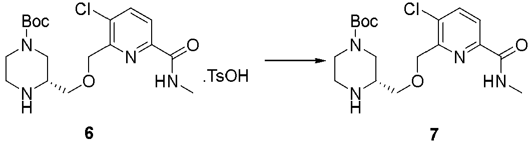

To a flask were added **compound 6** (26.0 g, 45.53 mmol, 1.0 eq.) and ethyl acetate (130 mL, 5 V), the mixture was stirred evenly, and saturated sodium bicarbonate (78 mL, 3 V) was added in portions to dissolution and clarification. The organic phase was separated and concentrated to afford **compound 7** (17.6 g, yield: 96.92%, purity: 96.7%).

### Example 6 Preparation of (R)-tert-butyl 9-(methylcarbamyl)-1,2,4a,5-tetrahydro-7H-pyrazino[2,1-c]pyrido[3,2-e][1,4]oxazepine-3(4H)-carboxylate (compound 8)

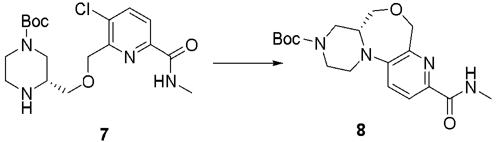

To a three-necked flask were added compound 7 (17.6 g, 44.12 mmol, 1.0 eq.), tris(dibenzylideneacetone)dipalladium (1.21 g, 1.32 mmol, 0.03 eq.), 2-dicyclohexylphosphino-2',6'-diisopropoxybiphenyl (1.24 g, 2.66 mol, 0.06 q.), caesium carbonate (17.25 g, 52.94 mmol, 1.2 eq.) and dioxane (141 mL, 8 V). Nitrogen replacement was carried out three times, and mixture was stirred at 100°C-110°C until the reaction was completed. Ethyl acetate and water were added, the organic phase was separated, and silicon-based metal scavenger MS001 was added to the organic phase. The mixture was stirred, filtered, and concentrated to afford **compound 8** (19.74 g, purity: 95.65%), which was directly used in the next reaction.

### Example 7 Preparation of (R)-N-methyl-1,2,3,4,4a,5-hexahydro-7H-pyrazino[2,1-c]pyrido[3,2-e][1,4]oxazepine-9-carboxamide dihydrochloride (compound 9c)

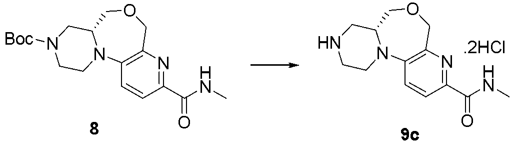

To a flask were added crude **compound 8** (19.74 g) and methanol (127 mL, 6.5 V), and the mixture was stirred for dissolution. 4N HCl/dioxane was dropwise added at room temperature, and the resulting mixture was stirred until the reaction was completed, and concentrated to afford **compound 9c** (13.85 g, two-step yield: 93.6%, purity: 98.48%).

### Example 8 Preparation of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide (compound 11)

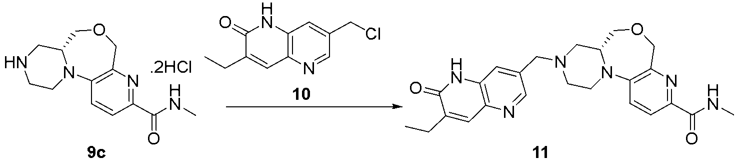

To a flask were added **compound 9c** (13.0 g, 38.78 mmol, 1.0 eq.), **compound 10** (8.63 g, 38.76 mmol, 1.0 eq.), sodium iodide (581 mg, 3.88 mmol, 0.1 eq.), N,N-dimethylformamide (43 mL, 5 V) and N,N-diisopropylethylamine (27.55 g, 213.16 mmol, 5.5 eq.), and the mixture was stirred at 50°C-60°C until the reaction was completed. Water was added, and the resulting mixture was filtered and dried to afford **compound 11** (16.08 g, yield: 92.4%, purity: 97.75%).

### Example 9 Preparation of (R)-3-((7-ethyl-6-oxo-5,6-dihydro-1,5-naphthyridin-3-yl)methyl)-N-methyl-1,2,3,4,4a,5-hexahydro-7H-pyrazino[2,1-c]pyrido[3,2-e][1,4]oxazepine-9-carboxamide fumaric acid salt (compound 12)

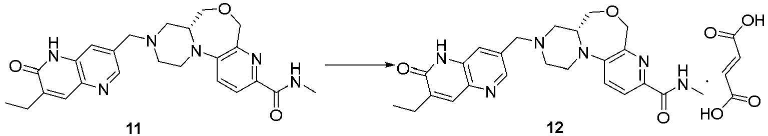

To a flask were added **compound 11** (15.48 g, 34.51 mmol, 1.0 eq.), fumaric acid (4.4 g, 37.91 mmol, 1.1 eq.), ethanol (464 mL, 30 V) and water (46 mL, 3 V), and the mixture was stirred at 85°C-95°C to dissolution and clarification, and filtered while hot. The filtrate was stirred at room temperature to precipitate a solid, and the mixture was filtered and dried to afford **compound 12** (16.60 g, yield: 85.2%, purity: 99.22%, chiral purity: 100%).

## Claims

1. A method for preparing a compound shown as formula 7a or a pharmaceutically acceptable salt thereof, **characterised by** comprising reacting a compound shown as formula 5a or a pharmaceutically acceptable salt thereof in the presence of carbon monoxide, a palladium catalyst and R₄-NH₂ to afford the compound shown as formula 7a or the pharmaceutically acceptable salt thereof, 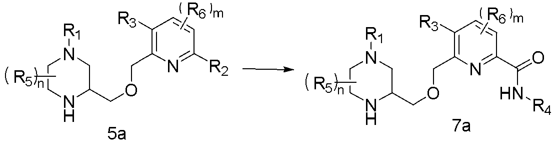
wherein R₁ is an amino protecting group;
R₂ is halogen;
R₃ is halogen;
R₄ is selected from hydrogen, C₁₋₆ alkyl, hydroxylated C₁₋₆ alkyl, or halogenated C₁₋₆ alkyl;
each R₅ is independently selected from halogen, C₁₋₆ alkyl, hydroxylated C₁₋₆ alkyl, halogenated C₁₋₆ alkyl, C₁₋₆ alkoxy, hydroxylated C₁₋₆ alkoxy, halogenated C₁₋₆ alkoxy, hydroxyl, cyano, or oxo;
each R₆ is independently selected from halogen, C₁₋₆ alkyl, hydroxylated C₁₋₆ alkyl, halogenated C₁₋₆ alkyl, C₁₋₆ alkoxy, hydroxylated C₁₋₆ alkoxy, halogenated C₁₋₆ alkoxy, hydroxyl, or cyano;
n is an integer selected from 0-6;
m is an integer selected from 0-2.

2. The preparation method according to claim 1, **characterised in that** the palladium catalyst is a divalent palladium catalyst, and preferably the palladium catalyst is selected from palladium acetate, palladium dichloride, tris(dibenzylideneacetone)dipalladium, bis(dibenzylideneacetone)palladium, palladium trifluoroacetate, palladium pivalate, tetrakis(triphenylphosphine)palladium, bis(triphenylphosphine)palladium chloride, bis(benzonitrile)palladium chloride, or bis(acetonitrile)dichloropalladium.

3. The preparation method according to claim 1, **characterised in that** the molar ratio of the compound shown as formula 5a to the palladium catalyst is 1 : 0.1 - 1 : 0.0001; preferably 1 : 0.01 - 1 : 0.0005; more preferably 1 : 0.01 - 1 : 0.001, and most preferably 1 : 0.005.

4. The preparation method according to claim 1, **characterised in that** the method comprises reacting the compound shown as formula 5a or the pharmaceutically acceptable salt thereof in the presence of a phosphine ligand to afford the compound shown as formula 7a or the pharmaceutically acceptable salt thereof.

5. The preparation method according to claim 4, **characterised in that** the phosphine ligand is selected from triphenylphosphine, tri(o-tolyl)phosphine, tri-tert-butylphosphine, tricyclohexylphosphine, 2-(dicyclohexylphosphino)biphenyl, 2-(di-tert-butylphosphino)biphenyl, 2-(dicyclohexylphosphino)-2'-methylbiphenyl, 2-(di-tert-butylphosphino)-2'-methylbiphenyl, 1,1'-bis(diphenylphosphino)ferrocene, (+)-2,2'-bis(diphenylphosphino)-1,1'-binaphthalene, 4,5-bis(diphenylphosphino)-9,9-dimethylxanthene, bis(2-diphenylphosphinophenyl)ether, 2-dicyclohexylphosphino-2',6'-diisopropoxybiphenyl, 2-dicyclohexylphosphino-2',4',6'-triisopropylbiphenyl, 2-(dicyclohexylphosphino)biphenyl, 2-(di-tert-butylphosphino)biphenyl, 2-dicyclohexylphosphino-2'-(N,N-dimethylamino)biphenyl, 2-dicyclohexylphosphino-2',6'-dimethoxybiphenyl, 2-(di-tert-butylphosphino)-3,6-dimethoxy-2'-4'-6'-triisopropyl-1,1'-biphenyl, dicyclohexyl(2',4',6'-triisopropyl-3,6-dimethoxy-[1,1'-biphenyl]-2-yl)phosphine, [(4-(N,N-dimethylamino)phenyl]di-tert-butylphosphine, 2-di-tert-butylphosphino-3,4,5,6-tetramethyl-2',4',6'-triisopropylbiphenyl, 1,2-bis(diphenylphosphino)ethane, 1,3-bis(diphenylphosphino)propane, or 1,4-bis(diphenylphosphino)butane, and preferably the phosphine ligand is 1,1'-bis(diphenylphosphino)ferrocene.

6. The preparation method according to claim 4, **characterised in that** the molar ratio of the compound shown as formula 5a to the phosphine ligand is 1 : 0.1 - 1 : 0.0001; preferably 1 : 0.01 - 1 : 0.0005; more preferably 1 : 0.01 - 1 : 0.001; and most preferably 1 : 0.005.

7. The preparation method according to claim 1, **characterised in that** the method comprises reacting the compound shown as formula 5a or the pharmaceutically acceptable salt thereof in the presence of a base to afford the compound shown as formula 7a or the pharmaceutically acceptable salt thereof.

8. The preparation method according to claim 7, **characterised in that** the base is selected from sodium hydroxide, sodium carbonate, caesium carbonate, triethylamine, sodium acetate or pyridine.

9. The preparation method according to claim 7, **characterised in that** the molar ratio of the compound shown as formula 5a to the base is 1 : 5 - 1 : 0.5, preferably 1 : 5 - 1 : 1, more preferably, and most preferably 1 : 2.

10. The preparation method according to claim 1, **characterised in that** the R₄ is selected from C₁₋₆ alkyl, hydroxylated C₁₋₆ alkyl, or halogenated C₁₋₆ alkyl, and preferably the R₄ is selected from methyl, ethyl, propyl, butyl, hydroxymethyl, hydroxyethyl, hydroxypropyl, hydroxybutyl, halomethyl, haloethyl, halopropyl, or halobutyl.

11. The preparation method according to claim 1, **characterised in that** R₁ is selected from C₁-C₆ alkylacyl, C₆-C₁₀ arylacyl, C₁-C₆ alkylsulfonyl, C₆-C₁₀ arylsulfonyl, C₁-C₆ alkoxycarbonyl, C₆-C₁₀ aryloxycarbonyl, or substituted or unsubstituted C₇-C₁₂ benzyl, preferably R₁ is selected from formyl, acetyl, benzoyl, tertbutoxycarbonyl (Boc), benzyloxycarbonyl (Cbz), 2,4-dimethoxybenzyl (DMB), p-methoxybenzyl (PMB) or benzyl (Bn), and more preferably R₁ is tertbutoxycarbonyl (Boc).

12. The preparation method according to claim 1, **characterised in that** the method comprises reacting a compound shown as formula 5b or a pharmaceutically acceptable salt thereof to afford a compound shown as formula 7b or a pharmaceutically acceptable salt thereof,

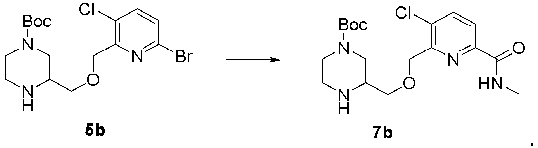

13. The preparation method according to claim 1, **characterised in that** the method comprises reacting a compound shown as formula 5 or a pharmaceutically acceptable salt thereof to afford a compound shown as formula 7 or a pharmaceutically acceptable salt thereof,

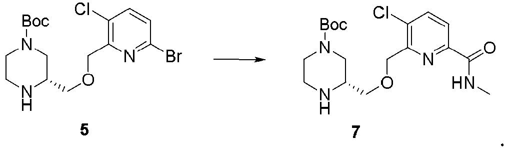

14. A method for preparing a compound shown as formula 8a or a pharmaceutically acceptable salt thereof, **characterised in that** the method comprises the method for preparing the compound shown as formula 7a or the pharmaceutically acceptable salt thereof according to claim 1, 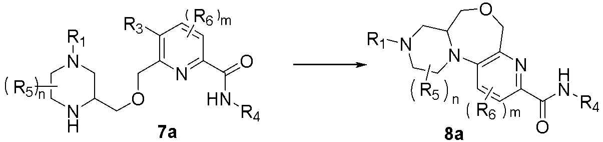 wherein, R₁, R₃, R₄, R₅, R₆, n and m are as defined in claim 1.

15. The preparation method according to claim 14, **characterised in that** the method comprises: reacting a compound shown as formula 7b or a pharmaceutically acceptable salt thereof to afford a compound shown as formula 8b or a pharmaceutically acceptable salt thereof,

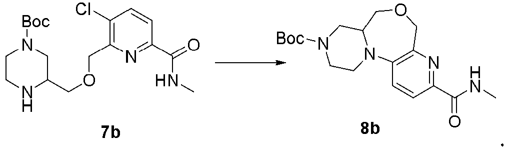

16. The preparation method according to claim 14, **characterised in that** the method comprises reacting a compound shown as formula 7 or a pharmaceutically acceptable salt thereof to afford a compound shown as formula 8 or a pharmaceutically acceptable salt thereof,

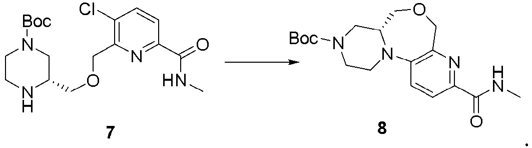

17. A method for preparing a compound shown as formula 11a or a pharmaceutically acceptable salt thereof, **characterised by** comprising
(1) preparing the compound shown as formula 8a or the pharmaceutically acceptable salt thereof according to the preparation method according to claim 14; and
(2) using the compound shown as formula 8a or the pharmaceutically acceptable salt thereof to prepare the compound shown as formula 11a or the pharmaceutically acceptable salt thereof; 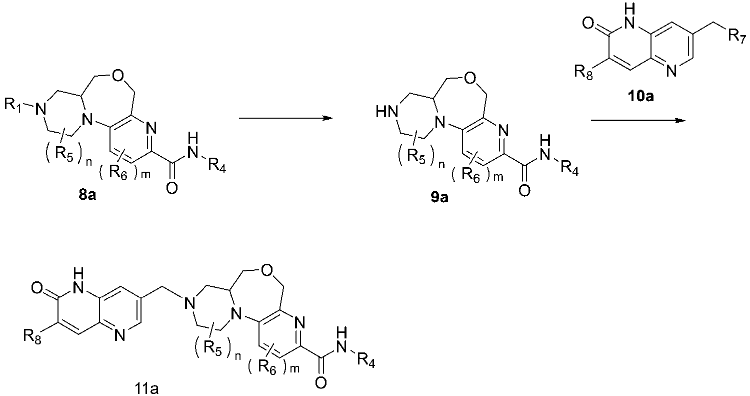
wherein, R₁, R₄, R₅, R₆, n and m are as defined in claim 1;
R₇ is halogen;
R₈ is selected from hydrogen, C₁₋₆ alkyl, hydroxylated C₁₋₆ alkyl, or halogenated C₁₋₆ alkyl, and preferably the R₈ is selected from methyl, ethyl, propyl, butyl, hydroxymethyl, hydroxyethyl, hydroxypropyl, hydroxybutyl, halomethyl, haloethyl, halopropyl, or halobutyl.

18. A method for preparing a compound shown as formula 11 or a pharmaceutically acceptable salt thereof, **characterised by** comprising
(1) preparing the compound shown as formula 8 or the pharmaceutically acceptable salt thereof according to the preparation method according to claim 16; and
(2) using the compound shown as formula 8 or the pharmaceutically acceptable salt thereof to prepare the compound shown as formula 11 or the pharmaceutically acceptable salt thereof;
